(19) Europäisches Patentamt
European Patent Office
Office européen des brevets

(11) Numéro de publication : **0 342 111 B1**

# (12) FASCICULE DE BREVET EUROPEEN

(45) Date de publication du fascicule du brevet :
**05.08.92 Bulletin 92/32**

(51) Int. Cl.⁵ : **A23K 1/16,** A23K 1/18, A61K 35/74

(21) Numéro de dépôt : **89401275.6**

(22) Date de dépôt : **05.05.89**

(54) **Procédé pour augmenter la productivité des truies.**

(30) Priorité : **09.05.88 FR 8806215**

(43) Date de publication de la demande :
**15.11.89 Bulletin 89/46**

(45) Mention de la délivrance du brevet :
**05.08.92 Bulletin 92/32**

(84) Etats contractants désignés :
**AT BE CH DE ES FR GB GR IT LI LU NL SE**

(56) Documents cités :
**EP-A- 0 296 051
DE-A- 2 622 982**

(56) Documents cités :
**CHEMICAL ABSTRACTS, vol. 106, no. 9, 2
mars 1987, page 527, résumé no. 66306t,
Columbus, Ohio, US; & CS-A-232 777 (M.DE-
DEK et al.) 15-01-1987
"Dictionnaire vidal", 1985, pages 142, OVP,
Paris, FR**

(73) Titulaire : **GUYOMARC'H NUTRITION
ANIMALE
TALHOUET en SAINT-NOLFF
F-56250 ELVEN (FR)**

(72) Inventeur : **Nguyen, Tan Hung
Le Porlair
F-56890 Saint Ave (FR)**

(74) Mandataire : **Ahner, Francis et al
CABINET REGIMBEAU, 26, avenue Kléber
F-75116 Paris (FR)**

EP 0 342 111 B1

## Description

La présente invention concerne un procédé pour augmenter la productivité des truies.

Dans tous les pays du monde, la mortalité infantile des porcelets est un des principaux facteurs qui handicapent la productivité des élevages porcins.

Les causes de cette mortalité des porcelets sont nombreuses et ne relèvent pas toujours de maladies infectieuses, comme le montrent les résultats de cette enquête citée par le magasine "L'Eleveur de Porcs" (Octobre 1982, n°139, page 23) :

### CAUSES DE MORTALITE AVANT SEVRAGE

#### (exprimées en % des porcelets)

| | |
|---|---|
| Morts-nés | 1,6 |
| Pendant la mise-bas | 4,5 |
| Développement insuffisant | 3,5 |
| Traumatisme-Ecrasement | 4,0 |
| Maladies digestives | 2,0 |
| Pneumonie | 0,6 |
| Septicémie | 0,9 |
| Malformations | 1,1 |
| Polyarthrite | 1,1 |
| Maladies diverses | 2,4 |
| Causes non déterminées | 0,5 |
| | ——— |
| | 22,3 |

Les spores végétatives du Bacillus IP 5832 sont commercialisées et utilisées depuis longtemps comme médicament anti-diarrhéique en médecine humaine sous la marque BACTISUBTIL® (Dictionnaire VIDAL).

FR-A-0 296 051 décrit l'utilisation d'un additif alimentaire non médicamenteux promoteur de croissance des animaux, ledit additif se caractérisant en ce qu'il comprend le Bacillus cereus souche IP 5832 et/ou ses spores végétatives.

La présente invention concerne l'utilisation non-thérapeutique du Bacillus IP 5832 et/ou de ses spores chez les truies reproductrices, comme moyen pour réduire les pertes de leurs porcelets et ainsi augmenter la productivité des truies.

Le Bacillus IP 5832 et/ou ses spores peuvent être administrés aux truies, par voie orale, selon toutes les pratiques couramment utilisées en élevage porcin : mélangés dans l'aliment, dispersés dans l'eau, conditionnés en comprimés, en gélules, etc.

Le Bacillus IP 5832 et/ou ses spores sont administrés aux truies avant la mise bas, et ensuite pendant l'allaitement des porcelets.

Les apports quotidiens de Bacillus IP 5832 et/ou de ses spores doivent être calculés selon le moyen d'administration. Ils correspondent, en moyenne, à l'ingestion normale par les truies d'un aliment complet pour truies contenant de 500 000 à 2 000 000 germes par gramme d'aliment.

Les exemples non limitatifs suivants illustrent l'invention.

## EXEMPLE N°1

28 truies gestantes, en parfaite santé, sont réparties en deux lots homogènes, 10 jours avant la date présumée de leur mise-bas.

Un lot (témoin) reçoit, à partir de cette date et, après la mise-bas, jusqu'au sevrage des porcelets à 26 jours, un aliment complet expérimental titrant 87,2% de matière sèche, 17,5% de protéine, 4,7% de matières grasses, 6,2% de cellulose et 6,9% de matières minérales.

Le deuxième lot reçoit, pendant la même période, un aliment de même composition de base, mais contenant du Bacillus IP 5832 à raison de 797 000 germes revivifiables par gramme d'aliment.

Les porcelets n'ont pas accès aux aliments des truies.

Les résultats moyens obtenus ont été les suivants :

|  | TEMOIN (1) | IP 5832 (2) | DIFFERENCE $\frac{(2)}{(1)}$ (%) |
|---|---|---|---|
| . NOMBRE DE TRUIES | 12* | 14 | - |
| . PORCELETS NES TOTAUX/TRUIE | 10,25 | 10,93 | - |
| . PORCELETS MORTS NES/TRUIE | 0,83 | 0,64 | - 22,89 |
| . PORCELETS NES VIVANTS/TRUIE | 9,42 | 10,29 | + 9,24 |
| . PORCELETS SEVRES /TRUIE | 8,33 | 9,43 | + 13,21 |
| . MORTALITE A LA NAISSANCE (%) | 8,10 | 5,86 | - 27,65 |
| . MORTALITE DES PORCELETS NES VIVANTS, DE LA NAISSANCE AU SEVRAGE (%) | 11,57 | 8,36 | - 27,74 |
| . PERTES TOTALES SUR PORCELETS NES TOTAUX (%) | 18,73 | 13,72 | - 26,75 |
| . CONSOMMATION D'ALIMENT PAR TRUIE (kg) | 147,0 | 142,6 | - |

* 2 truies éliminées de l'essai : 1 pour agalactie et 1 pour mort subite.

## EXEMPLE N°2

32 truies, cliniquement saines, sont réparties en deux lots homogènes, une semaine avant la date présumée de leur mise-bas.

Un premier lot reçoit, à partir de la répartition et pendant toute la durée de l'allaitement des porcelets (26 jours), un aliment complet du commerce contenant 86,5% de matière sèche, 17,6% de protéine, 3,7% de matières grasses, 5,9% de cellulose et 6,1% de matières minérales. Ce premier lot sert de témoin.

Le deuxième lot reçoit, pendant la même période, le même aliment de base mais supplémenté avec du Bacillus IP 5832 à raison de 1 200 000 germes revivifiables par gramme d'aliment.

Les porcelets n'ont pas accès aux aliments des truies.

Les résultats moyens obtenus ont été les suivants :

3

| | TEMOIN (A) | IP 5832 (B) | DIFFERENCE $\frac{(B)}{(A)}$ (%) |
|---|---|---|---|
| . NOMBRE DE TRUIES | 16 | 16 | - |
| . PORCELETS NES TOTAUX /TRUIE | 11,81 | 11,13 | - |
| . PORCELETS MORTS-NES /TRUIE | 1,25 | 0,38 | - 69,60 |
| . PORCELETS NES VIVANTS /TRUIE | 10,56 | 10,75 | + 1,80 |
| . PORCELETS SEVRES/TRUIE | 8,13 | 9,13 | + 12,30 |
| . MORTALITE A LA NAISSANCE (%) | 10,58 | 3,41 | - 67,77 |
| . MORTALITE DES PORCELETS NES VIVANTS, DE LA NAISSANCE AU SEVRAGE (%) | 23,01 | 15,07 | - 34,51 |
| . PERTES TOTALES SUR PORCELETS NES TOTAUX (%) | 31,16 | 17,97 | - 42,33 |
| . CONSOMMATION D'ALIMENT PAR TRUIE (Kg) | 134,6 | 135,0 | - |

Bien que les valeurs absolues des améliorations soient différentes dans les deux exemples, les tendances obtenues ont été identiques.

Les mécanismes d'action de cette nouvelle application du Bacillus IP 5832 et/ou de ses spores demeurent inconnus.

L'invention est d'autant plus originale que les truies dans les deux exemples cités ont été en parfaite santé, comme il a été précisé précédemment.

## Revendications

1. Procédé non-thérapeutique pour augmenter la productivité des truies, caractérisé par l'administration aux truies reproductrices, par voie orale, de Bacillus IP 5832 et/ou de ses spores.

2. Procédé selon la revendication 1, caractérisé en ce que le Bacillus IP 5832 et/ou ses spores sont administrés aux truies avant la mise-bas et pendant l'allaitement des porcelets.

3. Procédé selon l'une des revendications 1 et 2, caractérisé en ce que les apports quotidiens de Bacillus IP 5832 et/ou ses spores correspondent à l'ingestion normale par les truies d'un aliment complet pour truies contenant de 500 000 à 2 000 000 germes revivifiables par gramme d'aliment.

4. Aliments, ou tout autre produit, destinés aux truies, contenant du Bacillus IP 5832 et/ou ses spores, pour la mise en oeuvre du procédé selon l'une des revendications 1, 2 et 3.

## Patentansprüche

1. Nicht-therapeutisches Verfahren zur Steigerung der Produktivität von Mutterschweinen, gekennzeichnet durch die Verabreichung von Bazillus IP 5832 und/oder seinen Sporen an Zucht-Mutterschweine, auf oralem Wege.

2. Verfahren nach Anspruch 1, dadurch gekennzeichnet, daß Bazillus IP 5832 und/oder seine Sporen den Mutterschweinen vor dem Werfen und während des Säugens der Ferkel verabreicht werden.

3. Verfahren nach einem der Ansprüche 1 und 2, dadurch gekennzeichnet, daß die täglichen Anteile an Bazillus IP 5832 und/oder seiner Sporen der normalen Aufnahme der Mutterschweine eines vollständigen Nahrungsmittels für Mutterschweine mit einem Gehalt von 500000 bis 2 000 000 lebensfähigen Keimen pro Gramm Nahrungsmittel entsprechen.

4. Nahrungsmittel oder jegliches andere Produkt, bestimmt für Mutterschweine, enthaltend Bazillus IP 5832 und/oder seine Sporen, zur Durchführung des Verfahrens nach einem der Ansprüche 1, 2 und 3.

**Claims**

1. A non-therapeutic method for increasing the productivity of sows, wherein Bacillus IP 5832 and/or its spores is/are administered orally to breeding sows.

2. The method as claimed in claim 1, wherein Bacillus IP 5832 and/or its spores is/are administered to the sows before farrowing and during suckling of the piglets.

3. The method as claimed in one of claims 1 and 2, wherein the daily provision of Bacillus IP 5832 and/or its spores correspond to the normal intake by the sows of a complete feed for sows containing 500,000 to 2,000,000 revitalizable microorganisms per gram of feed.

4. Feeds, or any other product, intended for sows, containing Bacillus IP 5832 and/or its spores, for carrying out the method as claimed in one of claims 1, 2 and 3.